# EUROPEAN PATENT SPECIFICATION

(11) **EP 2 798 553 B1**
(45) Date of publication and mention of the grant of the patent: **09.02.2022**
(21) Application number: 12861498.9
(22) Date of filing: 26.12.2012
(51) Int. Cl.: G16B 50/40, G06F 16/903, G16B 30/00, G06F 21/62, G16H 10/60

(54) **APPARATUS AND METHOD FOR MANAGING GENETIC INFORMATION**
VORRICHTUNG UND VERFAHREN ZUR VERWALTUNG GENETISCHER INFORMATIONEN
APPAREIL ET PROCÉDÉ PERMETTANT DE GÉRER DES INFORMATIONS GÉNÉTIQUES

(30) Priority: 27.12.2011 KR 20110143958
(43) Date of publication of application: 05.11.2014
(73) Proprietor: Macrogen Inc., Geumcheon-gu Seoul 153-781 (KR); SNU R&DB Foundation, Seoul 151-742 (KR)
(72) Inventor: CHUNG, Hedoo, Seoul 151-851 (KR); SEO, Jeong-Sun, Seoul 135-972 (KR); RHEE, Hwanseok, Seoul 153-801 (KR)
(74) Representative: Krauns, Christian
(86) International application number: PCT/KR2012/011477
(87) International publication number: WO 2013/100572

(56) References cited:
- JP-A- 2003 242 154
- JP-A- 2007 172 202
- KR-A- 20040 051 748
- KR-B1- 101 035 959
- US-A1- 2005 043 964
- US-B1- 6 397 224
- "Data storage and DNA banking for biomedical research: technical, social and ethical issues", EUROPEAN JOURNAL OF HUMAN GENETICS, vol. 11, no. 12, 22 October 2003 (2003-10-22), pages 906-908, XP55200875, ISSN: 1018-4813, DOI: 10.1038/sj.ejhg.5201107
- Bartha Maria Knoppers ET AL: "The Babel of genetic data terminology", Nature Biotechnology, 8 August 2005 (2005-08-08), pages 925-927, XP55200880, Retrieved from the Internet: URL:http://www.nature.com/nbt/journal/v23/ n8/pdf/nbt0805-925.pdf [retrieved on 2015-07-08]

## Description

### [Technical Field]

The present invention generally relates to an apparatus and a method for managing genetic information.

### [Background Art]

Human genetic information is stored to a base sequence of about three billion bases composed of Adenine, Guanine, Cytosine, and Thymine. A technique for decoding an entire base sequence of a subject is commercially available. However, genes whose meanings are interpreted are only part of about thirty thousand genes encrypted by the base sequences.

Because genetic information encrypted by the base sequence is continuously interpreted, the subject having the decoded base sequence will repeatedly check his or her base sequence in order to determine whether or not to have base sequence of the newly interpreted gene.

However, because the genetic information of a certain person indicates genetic characteristic of the family as well as his or her genetic characteristic, an infringement such as hacking to the genetic information can cause serious damages to him or her and the family.

An information technology that extends to a mobile communication service, a financial service, and a medical service creates various technologies such as an encryption technology for protecting the security of the personal information from being infringed. However, the genetic information requires a more powerful protecting technology for the security than other personal information.

The US 2005/043964 A1 discloses a data processing system for the processing of patient data including person identifying data of a respective patient and corresponding health data, with a central system, which includes a database storing the health data, and with terminal devices, which are connected with the central station for the request of health data from the database and/or for the input of health data into the central database. The US 6,397,224 B1 discloses a system for anonymously linking a plurality of data records, each data record comprising a plurality of elements for identifying an associated individual, including a first identity reference encoding module configured to encode a first encoded identity reference from a first subset of the identifying elements of a data record; a second identity reference encoding module configured to encode a second encoded identity reference from a second subset of the identifying elements of the data record; and an anonymization code assignment module configured to assign to each of the first and second encoded identity references an identical anonymization code for anonymously representing the individual associated with the data record.

### [Disclosure]

### [Technical Problem]

Aspects of the present invention provide genetic information managing apparatuses methods for securely storing and searching for human genetic information

### [Technical Solution]

The problem of the present invention is solved by an apparatus for managing genetic information according to the respective independent claims 1 and 7 as well as by a method of managing genetic information according to the respective independent claim 6 and 9. The dependent claims refer to further advantageous developments of the present invention.

### [Advantageous Effects]

According to an embodiment of the present invention, if identification information for a plurality of base code groups of a subject is exactly identified, a base sequence of a subject can be exactly restored and searched. However, when the identification information for the base code groups of the subject is not exactly identified, the base sequence of the subject cannot be identified.

Further, when the subject wants to discard his or her own genetic information, the subject just needs to destroy identification information on a plurality of base code groups. Destroying the identification information can produce the same effect as deleting the genetic information in the database.

### [Description of Drawings]

FIG. 1 is a block diagram of a genetic information managing apparatus according to an embodiment of the present invention.
FIG. 2 is a flowchart of a method for storing genetic information according to an embodiment of the present invention.
FIG. 3 is a flowchart of a method for searching for genetic information according to an embodiment of the present invention.
FIG. 4 shows arrangement and division of a base sequence, and allocation and store of identifiers according to an embodiment of the present invention.

### [Mode for Invention]

In the following detailed description, only certain embodiments of the present invention have been shown and described, simply by way of illustration. As those skilled in the art would realize, the described embodiments may be modified in various different ways.

Accordingly, the drawings and description are to be regarded as illustrative in nature and not restrictive. Like reference numerals designate like elements throughout the specification.

Now, a genetic information managing apparatus and method according to an embodiment of the present invention is described with reference to the drawings.

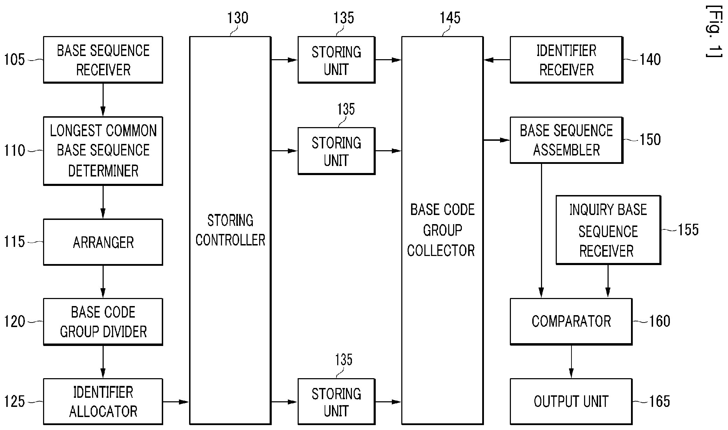

FIG. 1 is a block diagram of a genetic information managing apparatus according to an embodiment of the present invention.

Referring to FIG. 1, a genetic information managing apparatus according to an embodiment of the present invention includes a base sequence receiver 105, a longest common base sequence determiner 110, an arranger 115, a base code group divider 120, an identifier allocator 125, a storing controller 130, a plurality of storing units 135, an identifier receiver 140, a base code group collector 145, a base sequence assembler 150, an inquiry base sequence receiver 155, a comparator 160, and an output unit 165.

The base sequence receiver 105 receives a decoded base sequence of a subject.

The longest common base sequence determiner 110 compares the received base sequence with a standard base sequence and determines a longest common base sequence that corresponds to a longest interval from among common base sequence intervals.

The arranger 115 arranges the base sequence of the subject on the standard base sequence in accordance with the longest common base sequence that is determined by the longest common base sequence determiner 110.

The base code group divider 120 divides the base sequence arranged by the arranger 115 into a plurality of base code groups in accordance with a particular rule. The particular rule may correspond to a plurality of progressions.

The identifier allocator 125 allocates a plurality of identifiers to the base code groups that are divided by the base code group divider 120.

The storing controller 130 stores the base code groups to the storing units 135, respectively.

The storing units 135 may be physically or logically separated.

The identifier receiver 140 receives identification information composed of a plurality of identifiers through an input device from the subject.

The base code group collector 145 searches for a base code group corresponding to each identifier in the storing unit 135 corresponding to each identifier, and collects a plurality of base code groups that correspond to the identifiers, respectively.

The base sequence assembler 150 assembles the base code groups collected by the base code group collector 145 in accordance with the particular rule used for dividing the base sequence, and generates a base sequence of the subject corresponding to the identification information.

The inquiry base sequence receiver 155 receives an inquiry base sequence through the input device from the subject.

The comparator 160 compares the base sequence assembled by the base sequence assembler 150 with the inquiry base sequence received by the inquiry base sequence receiver 155, and determines whether the assembled base sequence includes the inquiry base sequence.

The output unit 165 outputs a comparison result of the comparator 160, and deletes the assembled base sequence in memory.

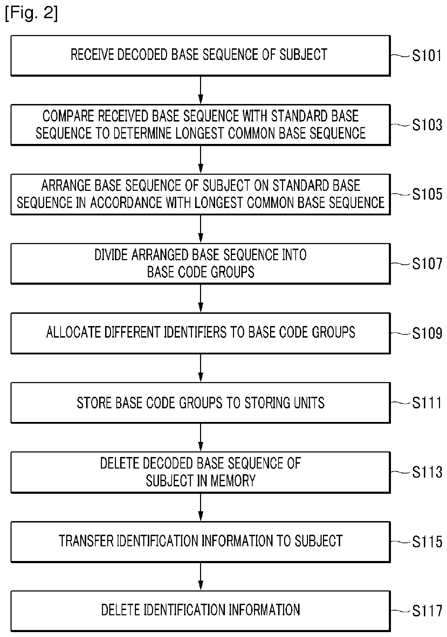

Next, a method for storing genetic information according to an embodiment of the present invention is described with reference to FIG. 2.

FIG. 2 is a flowchart of a method for storing genetic information according to an embodiment of the present invention.

Referring to FIG. 2, a base sequence receiver 105 receives a decoded base sequence of a subject (S101).

A longest common base sequence determiner 110 compares the received base sequence with a standard base sequence to determine a longest common base sequence that corresponds to a longest interval from among common base sequence intervals (S103). For example, when the base sequence of the subject4 is "...AAGCATCC..." and the standard base sequence is "...ATGCATGC...", the longest common base sequence is "GCAT".

An arranger 115 arranges the base sequence of the subject on the standard base sequence in accordance with the determined longest common base sequence (S105).

A base code group divider 120 divides the arranged base sequence into a plurality of base code groups in accordance with a particular rule (S107). The base code group divider 120 may extract the base code groups that correspond to a plurality of progressions respectively from the arranged base sequence. For example, when the base code group divider 120 generates two base code groups, the base code group divider 120 may extract one base code group corresponding to a progression 2n and another base code group corresponding to a progression 2n-1 from the base sequence, where n is a natural number. In other words, when the base sequence of the subject is "...AAGCATCC...", the base code group divider 120 may generate the base code group "...XAXCXTXC..." corresponding to the progression 2n and the base code group "...AXGXAXCX..." corresponding to the progression 2n-1. While it has been exemplified that the two base code groups are generated from the base sequence by using the progression 2n for extracting odd numbered elements and the progression 2n-1 for extracting even numbered elements, a plurality of progressions used by the base code group divider 120 may or may not have a duplicated element. Further, the base code group divider 120 may use more complicated progressions to divide more base code groups, thereby improving the security for the genetic information.

The identifier allocator 125 allocates different identifiers to the base code groups (S109). The identifier is used for a password or certification information for inquiring the genetic information. For example, the identifier allocator 125 may allocate an identifier 5 to the base code group "...XAXCXTXC..." corresponding to the progression 2n and an identifier 8 to the base code group "...AXGXAXCX..." corresponding to the progression 2n-1. The identifier allocator 125 may generate a plurality of identifiers by using a table of random numbers or a random number generator, and allocate the identifiers to the base code groups.

The storing controller 130 stores the base code groups to the storing units 135, respectively (Sill). The storing controller 130 may store each base code group to a corresponding storing unit 135 in association with a corresponding identifier. The storing unit 135 may be configured as a plurality of storing units that may be physically or logically divided. In this case, the storing controller 130 may store the base code group "...XAXCXTXC..." corresponding to the progression 2n to a storing unit, which corresponds to the progression 2n from among the storing units 135, in association with the identifier 5. Further, the storing controller 130 may store the base code group "...AXGXAXCX..." corresponding to the progression 2n-1 to a storing unit, which corresponds to the progression 2n-1 from among the storing units 135, in association with the identifier 8.

After the base code groups are stored, the base sequence receiver 105 deletes the decoded base sequence of the subject in memory (S113).

The identifier allocator 125 transfers identification information through an output device to the subject (S115).

On receiving an acknowledge message for the identification information through the input device from the subject, the identifier allocator 125 deletes the identification information in the memory (S117). As such, because the identifier allocator 125 deletes the identification information, the genetic information managing apparatus no longer has information about mappings between identifiers and subjects. Instead, the base code groups are stored to the storing units 135 in association with the identifiers.

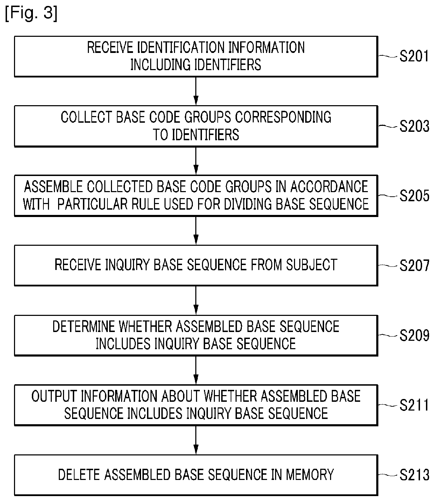

Next, a method for searching for genetic information according to an embodiment of the present invention is described with reference to FIG. 3.

FIG. 3 is a flowchart of a method for searching for genetic information according to an embodiment of the present invention.

Referring to FIG. 3, an identifier receiver 140 receives identification information including a plurality of identifiers through an input device from a subject (S201).

The base code group collector 145 searches for a base code group corresponding to each identifier in the storing unit 135 corresponding to each identifier, and collects a plurality of base code groups corresponding to the identifiers (S203).

The base sequence assembler 150 assembles the collected base code groups in accordance with a particular rule used for dividing the base sequence to generate a base sequence of the subject corresponding to the identification information (S205). The base sequence assembler 150 may assemble the base code groups in accordance with a plurality of progressions used for dividing the base sequence.

An inquiry base sequence receiver 155 receives an inquiry base sequence through the input device from the subject (S207). The inquiry base sequence is a base sequence for specific genetic information, and may be, for example, a base sequence for genetic information that can cause a specific disease.

A comparator 160 compares the base sequence assembled by the base sequence assembler 150 with the inquiry base sequence received by the inquiry base sequence receiver 155 to determine whether the assembled base sequence includes the inquiry base sequence (S209).

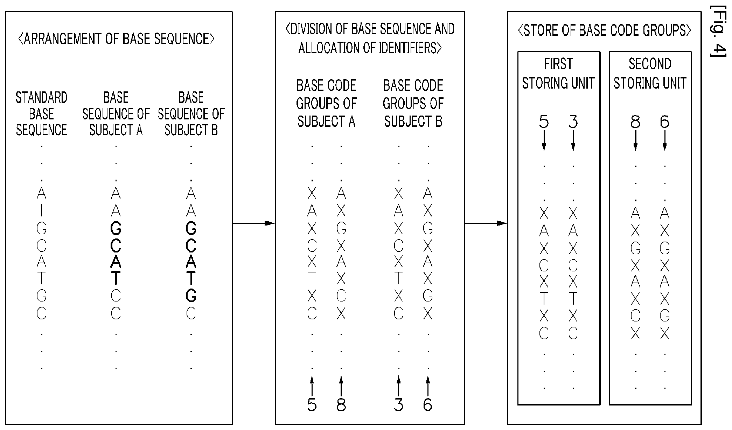

The output unit 165 outputs information about whether the assembled base sequence includes the inquiry base sequence (S211), and then deletes the assembled base sequence in memory (S213) FIG. 4 shows arrangement and division of a base sequence, and allocation and store of identifiers according to an embodiment of the present invention.

A base sequence "...AAGCATCC..." of a subject A and a base sequence "...AAGCATGC..." of a subject B are exemplified in FIG. 4. Further, it is assumed that a standard base sequence is "...ATGCATGC..." and two identifiers are used for securely storing a base sequence of a subject.

In this case, a longest common base sequence for the base sequence of the subject A is "GCAT", and the base sequence of the subject A is arranged in accordance with the longest common base sequence "GCAT".

Next, the base sequence "...AAGCATCC..." of the subject A is divided into a base code group "...XAXCXTXC..." corresponding to a progression 2n and a base code group "...AXGXAXCX..." corresponding to a progression 2n-1. Identifiers 5 and 8 are allocated to the two base code groups, respectively.

The base code group "...XAXCXTXC..." corresponding to the progression 2n is stored to a first storing unit in association with the identifier 5, and the base code group "...AXGXAXCX..." corresponding to the progression 2n-1 is stored to a second storing unit in association with the identifier 8.

Further, a longest common base sequence for the base sequence of the subject B is "GCATG", and the base sequence of the subject B is arranged in accordance with the longest common base sequence "GCATG" .

Next, the base sequence "...AAGCATGC..." of the subject B is divided into a base code group "...XAXCXTXC..." corresponding to the progression 2n and a base code group "...AXGXAXGX..." corresponding to the progression 2n-1. Identifiers 3 and 6 are allocated to the two base code groups, respectively.

The base code group "...XAXCXTXC..." corresponding to the progression 2n is stored to the first storing unit in association with the identifier 5, and the base code group "...AXGXAXGX..." corresponding to the progression 2n-1 is stored to the second storing unit in association with the identifier 8.

Therefore, when an identifier receiver 140 receives identification information corresponding to the identifiers 5 and 8 from the subject A, a base code group collector 145 can collect the base code group corresponding to the identifier 5 and the base code group corresponding to the identifier 8 from the storing units 135. A base sequence assembler 150 assembles the two base code groups according to a particular rule used for dividing the base sequence, i.e., the progressions 2n and 2n-1, to generate the base sequence of the subject A. A comparator 160 can compare the base sequence of the subject A, which is assembled by the base sequence assembler 150, with the inquiry base sequence receiver which is received by the inquiry base sequence receiver 155 to determine whether the base sequence of the subject A includes the inquiry base sequence.

As described above, according to an embodiment of the present invention, if identification information for a plurality of base code groups of a subject is exactly identified, entire base sequence of the subject can be restored and searched. However, when the identification information for the base code groups of the subject is not exactly identified, the base sequence of the subject cannot be identified even if information on the entire base sequence that is stored in a database is leaked by a hacking. In other words, the base sequence of the subject cannot be identified because all combinations of base code groups correspond to base sequences that can be biologically existed.

Further, when the subject wants to discard his or her own genetic information, the subject just needs to destroy identification information on a plurality of base code groups. In other words, because destroying the identification information can produce the same effect as deleting the genetic information in the database, powerful protecting technology for the security can be provided.

An apparatus and a method for managing genetic information according to an embodiment of the present invention can be combined with a general encryption scheme.

## Claims

1. An apparatus for managing genetic information, comprising:
a base sequence receiver (105) configured to receive a decoded base sequence of a subject; **characterized by**:
a longest common base sequence determiner (110) configured to determine a longest common base sequence that corresponds to common base sequence intervals between the base sequence and a standard base sequence; and
an arranger (115) configured to arrange the base sequence on the standard base sequence in accordance with the longest common base sequence, wherein a
base code group divider (120) divides the arranged base sequence into a plurality of base code groups, the base code group divider (120) configured to divide the base sequence into the plurality of base code groups in accordance with a plurality of progressions, wherein the plurality of progressions correspond to the plurality of base code groups, respectively, and each of the plurality of progressions is used for extracting a corresponding base code group from the base sequence;
an identifier allocator (125) configured to allocate a plurality of identifiers to the plurality of base code groups, respectively;
a plurality of storing units (135); and
a storing controller (130) configured to store the plurality of base code groups to the plurality of storing units (135) respectively, in association with corresponding identifiers;
wherein the base sequence receiver (105) deletes the base sequence of the subject in memory after storing the plurality of base code groups to the plurality of storing units (135), and
wherein the identifier allocator (125) deletes the plurality of identifiers in memory after transferring identification information corresponding to the plurality of identifiers to the subject.

2. The apparatus of claim 1, wherein the longest common base sequence corresponds to a longest interval from among the common base sequence intervals.

3. The apparatus of claim 1, wherein the plurality of progressions have a duplicated element.

4. The apparatus of claim 1, wherein the plurality of progressions have no duplicated element.

5. The apparatus of claim 1, wherein the identifier allocator (125) generates the plurality of identifiers by generating random numbers, and allocates the plurality of identifiers to the plurality of base code groups, respectively.

6. A method of managing genetic information of a subject carried out by a genetic information managing apparatus,
the method comprising following steps:
(S101) a receiving of a decoded base sequence of the subject; **characterized by**:
(S103) a determining of a longest common base sequence that corresponds to common base sequence intervals between the base sequence and a standard base sequence;
(S105) an arranging of the base sequence on the standard base sequence in accordance with the longest common base sequence;
(S107) a dividing of the base sequence into a plurality of base code groups in accordance with a plurality of progressions, wherein (S107) the dividing of the base sequence includes a dividing of the arranged base sequence into the plurality of base code groups, the plurality of progressions correspond to the plurality of base code groups, respectively, and each of the plurality of progressions is used for extracting a corresponding base code group from the base sequence;
(S109) an allocating of a plurality of identifiers to the plurality of base code groups, respectively; and
(S111) an storing the plurality of base code groups to the plurality of storing units (135) respectively, in association with corresponding identifiers;
S113) a deleting of the base sequence of the subject in
memory after storing the plurality of
base code groups to the plurality of storing units (135), and
(S117) a deleting of the plurality of identifiers in memory after transferring identification information corresponding to the plurality of identifiers to the subject (S115).

7. An apparatus for managing genetic information, comprising:
an identifier receiver (140) configured to receive identification information of a subject, the identification information including a plurality of identifiers; **characterized by**:
a plurality of storing units (135) configured to store a plurality of base code groups in association with a plurality of identifiers, the plurality of base code groups being formed by dividing a base sequence;
a base code group collector (145) configured to collect a plurality of base code groups that correspond to the plurality of identifiers of the identification information, respectively;
a base sequence assembler (150) configured to assemble the collected base code groups in accordance with a plurality of progressions used for dividing the base sequence to generate a base sequence of a subject, wherein the plurality of progressions correspond to the plurality of base code groups, respectively, and each of the plurality of progressions is used for extracting a corresponding base code group from the base sequence;
an inquiry base sequence receiver (155) configured to receive an inquiry base sequence;
an comparator (160) configured to compare the base sequence of the subject with the inquiry base sequence to determine whether the base sequence of the subject includes the inquiry base sequence;and
an output unit (165) configured to output information about whether the base sequence of the subject includes the inquiry base sequence and delete the base sequence of the subject in memory

8. The apparatus of claim 7, wherein the output unit (165) deletes the base sequence of the subject in a memory after outputting the information about whether the base sequence of the subject includes the inquiry base sequence.

9. A method of managing genetic information of a subject carried out by a genetic information managing apparatus,
the method comprising following steps:
(S201) a receiving of identification information of the subject, the identification information including a plurality of identifiers; **characterized by**:
a searching for a base code group corresponding to each identifier in a plurality of storing units (135) and (S203) and a collecting of a plurality of base code groups that correspond to the plurality of identifiers, respectively;
(S205) an assembling of the plurality of base code groups in accordance with a plurality of progressions used for dividing a base sequence to generate a base sequence of the subject, wherein the plurality of progressions correspond to the plurality of base code groups, respectively, and each of the plurality of progressions is used for extracting a corresponding base code group from the base sequence;
(S207) a receiving of an inquiry base sequence;
(S209) a comparing of the base sequence of the subject with the inquiry base sequence to determine whether the base sequence of the subject includes the inquiry base sequence;
(S211) an outputting of information about whether the base sequence of the subject includes the inquiry base sequence; and
(S213) a deleting of the base sequence of the subject in memory.

## Patentansprüche

1. Vorrichtung zur Verwaltung genetischer Informationen, welche aufweist:
einen Empfänger (105) für Basensequenzen, der so ausgestaltet ist, dass er eine dekodierte Basensequenz eines Patienten empfängt; **gekennzeichnet durch**:
einen Bestimmer (110) für längste gemeinsame Basensequenzen, der so ausgestaltet ist, dass er eine längste gemeinsame Basensequenz bestimmt, die gemeinsamen Basensequenzintervallen zwischen der Basensequenz und einer Standardbasensequenz entspricht; und
einen Anordner (115), der so ausgestaltet ist, dass er die Basensequenz auf der Standardbasensequenz in Übereinstimmung mit der längsten gemeinsamen Basensequenz anordnet, wobei ein Basencode-Gruppen-Teiler (120) die angeordnete Basensequenz in eine Vielzahl von Basencode-Gruppen unterteilt,
wobei der Basencode-Gruppen-Teiler (120) so ausgestaltet ist, dass er die Basensequenz in die Vielzahl der Basencode-Gruppen in Übereinstimmung mit einer Vielzahl von Progressionen unterteilt, wobei die Vielzahl der Progressionen jeweils der Vielzahl der Basencode-Gruppen entspricht und wobei jede von der Vielzahl der Progressionen zum Extrahieren einer entsprechenden Basencode-Gruppe aus der Basensequenz verwendet wird;
einen Zuweiser (125) für Identifikatoren, der so ausgestaltet ist, dass er eine Vielzahl an Identifikatoren jeweils der Vielzahl der Basencode-Gruppen zuweist;
eine Vielzahl an Speichereinheiten (135); und
eine Speichersteuerung (130), die so ausgestaltet ist, dass sie die Vielzahl der Basencode-Gruppen in der Vielzahl der Speichereinheiten (135) jeweils in Verbindung mit entsprechenden Identifikatoren speichert;
wobei der Empfänger (105) für Identifikatoren die Basensequenz des Patienten in einem Speicher löscht, nachdem er die Vielzahl der Basencode-Gruppen in der Vielzahl der Speichereinheiten (135) gespeichert hat, und
wobei der Zuweiser (125) für Identifikatoren die Vielzahl der Identifikatoren in einem Speicher löscht, nachdem er Identifikator-Informationen, die der Vielzahl der Identifikatoren entsprechen, an den Patienten übertragen hat.

2. Vorrichtung nach Anspruch 1, bei der die längste gemeinsame Basensequenz einem längsten Intervall unter den gemeinsamen Basensequenzintervallen entspricht.

3. Vorrichtung nach Anspruch 1, bei der die Vielzahl der Progressionen ein dupliziertes Element aufweist.

4. Vorrichtung nach Anspruch 1, bei der die Vielzahl der Progressionen kein dupliziertes Element aufweist.

5. Vorrichtung nach Anspruch 1, bei der der Zuweiser (125) für Identifikatoren die Vielzahl der Identifikatoren durch ein Erzeugen von Zufallszahlen erzeugt und er die Vielzahl der Identifikatoren jeweils der Vielzahl der Basencode-Gruppen zuweist.

6. Verfahren zur Verwaltung genetischer Informationen eines Patienten, das von einer Vorrichtung zur Verwaltung genetischer Informationen durchgeführt wird, wobei das Verfahren die folgenden Schritte umfasst:
(S101) ein Empfangen einer entschlüsselten Basensequenz des Patienten; **gekennzeichnet durch**:
(S103) ein Bestimmen einer längsten gemeinsamen Basensequenz, die gemeinsamen Basensequenzintervallen zwischen der Basensequenz und einer Standardbasensequenz entspricht;
(S105) ein Anordnen der Basensequenz auf der Standardbasensequenz in Übereinstimmung mit der längsten gemeinsamen Basensequenz;
(S107) ein Aufteilen der Basensequenz in eine Vielzahl von Basencode-Gruppen gemäß einer Vielzahl an Progressionen, wobei (S107) das Aufteilen der Basensequenz ein Aufteilen der angeordneten Basensequenz in die Vielzahl der Basencode-Gruppen umfasst, wobei die Vielzahl der Progressionen jeweils der Vielzahl der Basencode-Gruppen entspricht und wobei jeder von der Vielzahl der Progressionen zum Extrahieren einer entsprechenden Basencode-Gruppe aus der Basensequenz verwendet wird;
(S109) ein Zuordnen einer Vielzahl an Identifikatoren an die Vielzahl der Basencode-Gruppen; und
(S111) ein Speichern der Vielzahl der Basencode-Gruppen in der Vielzahl der Speichereinheiten (135) jeweils in Verbindung mit entsprechenden Identifikatoren;
(S113) ein Löschen der Basensequenz des Patienten im Speicher nach einem Speichern der Vielzahl der Basencode-Gruppen in der Vielzahl der Speichereinheiten (135), und
(S117) ein Löschen der Vielzahl der Identifikatoren in einem Speicher nach (S115) einem Übertragen von Identifikator-Informationen, die der Vielzahl der Identifikatoren entsprechen, an den Patienten.

7. Vorrichtung zur Verwaltung genetischer Informationen, welche aufweist:
einen Empfänger (140) für Identifikatoren, der so ausgestaltet ist, dass er Identifikator-Informationen eines Patienten empfängt, wobei die Identifikator-Informationen eine Vielzahl an Identifikatoren enthalten; **gekennzeichnet durch**:
eine Vielzahl an Speichereinheiten (135), die so ausgestaltet sind, dass sie eine Vielzahl an Basencode-Gruppen in Verbindung mit einer Vielzahl an Identifikatoren speichern, wobei die Vielzahl der Basencode-Gruppen durch ein Unterteilen einer Basensequenz gebildet worden ist;
einen Sammler (145) für Basencode-Gruppen, der so ausgestaltet ist, dass er eine Vielzahl an Basencode-Gruppen sammelt, die jeweils der Vielzahl der Identifikatoren der Identifikator-Informationen entsprechen;
einen Zusammensetzer (150) für Basensequenzen, der so ausgestaltet ist, dass er die gesammelten Basencode-Gruppen in Übereinstimmung mit einer Vielzahl an Progressionen zusammensetzt, die zum Teilen der Basensequenz verwendet worden ist, um eine Basensequenz eines Patienten zu erzeugen, wobei die Vielzahl der Progressionen jeweils der Vielzahl der Basencode-Gruppen entspricht und wobei jeder von der Vielzahl der Progressionen zu einem Extrahieren einer entsprechenden Basencode-Gruppe aus der Basensequenz verwendet worden ist;
einen Empfänger (155) für Anfrage-Basensequenzen, der so ausgestaltet ist, dass er eine Anfrage-Basensequenz empfängt;
einen Komparator (160), der so ausgestaltet ist, dass er die Basensequenz des Patienten mit der Anfrage-Basensequenz vergleicht, um zu bestimmen, ob die Basensequenz des Patienten die Anfrage-Basensequenz enthält; und
eine Ausgabeeinheit (165), die so ausgestaltet ist, dass sie Informationen darüber ausgibt, ob die Basensequenz des Patienten die Anfrage-Basensequenz enthält, und sie die Basensequenz des Patienten in einem Speicher löscht.

8. Vorrichtung nach Anspruch 7, wobei die Ausgabeeinheit (165) die Basensequenz des Patienten in einem Speicher löscht, nachdem sie die Information darüber ausgegeben hat, ob die Basensequenz des Patienten die Abfrage-Basensequenz enthält.

9. Verfahren zur Verwaltung genetischer Informationen eines Patienten, das von einer Vorrichtung zur Verwaltung genetischer Informationen durchgeführt wird, wobei das Verfahren die folgenden Schritte umfasst:
(S201) ein Empfangen von Identifikator-Informationen des Patienten, wobei die Identifikator-Informationen eine Vielzahl an Identifikatoren enthalten; **gekennzeichnet durch**:
(S201) ein Suchen nach einer Basencode-Gruppe, die jeweils einem Identifikator in einer Vielzahl an Speichereinheiten (135) entspricht, und (S203) ein Sammeln einer Vielzahl an Basencode-Gruppen, die jeweils der Vielzahl der Identifikatoren entsprechen;
(S205) ein Zusammensetzen der Vielzahl der Basencode-Gruppen in Übereinstimmung mit einer Vielzahl an Progressionen, die zum Teilen einer Basensequenz verwendet werden, um eine Basensequenz des Patienten zu erzeugen, wobei die Vielzahl der Progressionen jeweils der Vielzahl der Basencode-Gruppen entspricht, und wobei jede von der Vielzahl der Progressionen zum Extrahieren einer entsprechenden Basencode-Gruppe aus der Basensequenz verwendet wird;
(S207) ein Empfangen einer Abfrage-Basensequenz;
(S209) ein Vergleichen der Basensequenz des Patienten mit der Anfrage-Basensequenz, um zu bestimmen, ob die Basensequenz des Patienten die Anfrage-Basensequenz enthält;
(S211) ein Ausgeben von Information darüber, ob die Basensequenz des Patienten die Anfrage-Basensequenz enthält; und
(S213) ein Löschen der Basensequenz des Patienten im Speicher.

## Revendications

1. Appareil pour gérer des informations génétiques, comprenant :
un récepteur de séquence de base (105) configuré pour recevoir une séquence de base décodée d'un sujet ;
**caractérisé par** :
un déterminateur de séquence de base commune la plus longue (110) configuré pour déterminer une séquence de base commune la plus longue qui correspond à des intervalles de séquence de base commune entre la séquence de base et une séquence de base standard ; et
un arrangeur (115) configuré pour arranger la séquence de base sur la séquence de base standard en conformité avec la séquence de base commune la plus longue, dans lequel un diviseur de groupe de code de base (120) divise la séquence de base arrangée en une pluralité de groupes de code de base, le diviseur de groupe de code de base (120) étant configuré pour diviser la séquence de base en la pluralité de groupes de code de base en conformité avec une pluralité de progressions, dans lequel la pluralité de progressions correspond à la pluralité de groupes de code de base, respectivement, et chacune de la pluralité de progressions est utilisée pour extraire un groupe de code de base correspondant à partir de la séquence de base ;
un attributeur d'identifiants (125) configuré pour attribuer une pluralité d'identifiants à la pluralité de groupes de code de base, respectivement ;
une pluralité d'unités de stockage (135) ; et
un dispositif de commande de stockage (130) configuré pour stocker la pluralité de groupes de code de base dans la pluralité d'unités de stockage (135) respectivement, en association avec des identifiants correspondants ;
dans lequel le récepteur de séquence de base (105) supprime la séquence de base du sujet en mémoire après stockage de la pluralité de groupes de code de base dans la pluralité d'unités de stockage (135), et
dans lequel l'attributeur d'identifiants (125) supprime la pluralité d'identifiants en mémoire après transfert d'informations d'identification correspondant à la pluralité d'identifiants au sujet.

2. Appareil selon la revendication 1, dans lequel la séquence de base commune la plus longue correspond à un intervalle le plus long parmi les intervalles de séquence de base commune.

3. Appareil selon la revendication 1, dans lequel la pluralité de progressions ont un élément dupliqué.

4. Appareil selon la revendication 1, dans lequel la pluralité de progressions n'ont pas d'élément dupliqué.

5. Appareil selon la revendication 1, dans lequel l'attributeur d'identifiants (125) génère la pluralité d'identifiants par génération de nombres aléatoires, et attribue la pluralité d'identifiants à la pluralité de groupes de code de base, respectivement.

6. Procédé de gestion d'informations génétiques d'un sujet effectué par un appareil de gestion d'informations génétiques,
le procédé comprenant les étapes suivantes :
(S101) une réception d'une séquence de base décodée du sujet ; **caractérisé par** :
(S103) une détermination d'une séquence de base commune la plus longue qui correspond à des intervalles de séquence de base commune entre la séquence de base et une séquence de base standard ;
(S105) un agencement de la séquence de base sur la séquence de base standard en conformité avec la séquence de base commune la plus longue ;
(S107) une division de la séquence de base en une pluralité de groupes de code de base en conformité avec une pluralité de progressions, dans lequel (S107) la division de la séquence de base comporte une division de la séquence de base arrangée en la pluralité de groupes de code de base, la pluralité de progressions correspond à la pluralité de groupes de code de base, respectivement, et chacune de la pluralité de progressions est utilisée pour extraire un groupe de code de base correspondant à partir de la séquence de base ;
(S109) une attribution d'une pluralité d'identifiants à la pluralité de groupes de code de base, respectivement ; et
(S111) un stockage de la pluralité de groupes de code de base dans la pluralité d'unités de stockage (135) respectivement, en association avec des identifiants correspondants ;
(S113) une suppression de la séquence de base du sujet en mémoire après stockage de la pluralité de groupes de code de base dans la pluralité d'unités de stockage (135), et
(S117) une suppression de la pluralité d'identifiants en mémoire après transfert d'informations d'identification correspondant à la pluralité d'identifiants au sujet (S115).

7. Appareil pour gérer des informations génétiques, comprenant :
un récepteur d'identifiant (140) configuré pour recevoir des informations d'identification d'un sujet, les informations d'identification comportant une pluralité d'identifiants ; **caractérisé par** :
une pluralité d'unités de stockage (135) configurées pour stocker une pluralité de groupes de code de base en association avec une pluralité d'identifiants, la pluralité de groupes de code de base étant formés par division d'une séquence de base ;
un collecteur de groupes de code de base (145) configuré pour collecter une pluralité de groupes de code de base qui correspondent à la pluralité d'identifiants des informations d'identification, respectivement ;
un assembleur de séquence de base (150) configuré pour assembler les groupes de code de base collectés en conformité avec une pluralité de progressions utilisées pour diviser la séquence de base afin de générer une séquence de base d'un sujet, dans lequel la pluralité de progressions correspondent à la pluralité de groupes de code de base, respectivement, et chacune de la pluralité de progressions est utilisée pour extraire un groupe de code de base correspondant à partir de la séquence de base ;
un récepteur de séquence de base d'interrogation (155) configuré pour recevoir une séquence de base d'interrogation ;
un comparateur (160) configuré pour comparer la séquence de base du sujet à la séquence de base d'interrogation afin de déterminer si la séquence de base du sujet comporte la séquence de base d'interrogation ; et
une unité de sortie (165) configurée pour fournir en sortie des informations concernant le fait que la séquence de base du sujet comporte ou non la séquence de base d'interrogation et supprimer la séquence de base du sujet en mémoire.

8. Appareil selon la revendication 7, dans lequel l'unité de sortie (165) supprime la séquence de base du sujet dans une mémoire après fourniture en sortie des informations concernant le fait que la séquence de base du sujet comporte ou non la séquence de base d'interrogation.

9. Procédé de gestion d'informations génétiques d'un sujet effectué par un appareil de gestion d'informations génétiques,
le procédé comprenant les étapes suivantes :
(S201) une réception d'informations d'identification du sujet, les informations d'identification comportant une pluralité d'identifiants ; **caractérisé par** :
une recherche d'un groupe de code de base correspondant à chaque identifiant dans une pluralité d'unités de stockage (135) et (S203) une collecte d'une pluralité de groupes de code de base qui correspondent à la pluralité d'identifiants, respectivement ;
(S205) un assemblage de la pluralité de groupes de code de base en conformité avec une pluralité de progressions utilisées pour diviser une séquence de base afin de générer une séquence de base du sujet, dans lequel la pluralité de progressions correspondent à la pluralité de groupes de code de base, respectivement, et chacune de la pluralité de progressions est utilisée pour extraire un groupe de code de base correspondant à partir de la séquence de base ;
(S207) une réception d'une séquence de base d'interrogation ;
(S209) une comparaison de la séquence de base du sujet à la séquence de base d'interrogation pour déterminer si la séquence de base du sujet comporte la séquence de base d'interrogation ;
(S211) une fourniture en sortie d'informations concernant le fait que la séquence de base du sujet comporte ou non la séquence de base d'interrogation ; et
(S213) une suppression de la séquence de base du sujet en mémoire.
